# EUROPÄISCHE PATENTSCHRIFT

(11) **EP 0 770 358 B1**
(45) Veröffentlichungstag und Bekanntmachung des Hinweises auf die Patenterteilung: **14.06.2000**
(21) Anmeldenummer: 96116934.9
(22) Anmeldetag: 22.10.1996
(51) Int. Cl.: A61B 17/28, A61B 17/34

(54) **Operationswerkzeug**
Medical operation tool
Outil médical d'opération

(30) Priorität: 26.10.1995 DE 29516958 U
(43) Veröffentlichungstag der Anmeldung: 02.05.1997
(73) Patentinhaber: Hariri, Bahman, 47877 Willich (DE)
(72) Erfinder: Bahman, Hariri, 47877 Willich (DE); Breitmar, Paul, 47877 Willich (DE)
(74) Vertreter: Stark, Walter, Dr.-Ing.

(56) Entgegenhaltungen:
- WO-A-89/10093
- WO-A-91/08709
- WO-A-94/05224
- DE-A- 4 115 548
- DE-U- 9 105 399
- FR-A- 2 508 791
- US-A- 2 644 455
- US-A- 4 889 118

## Beschreibung

Die Erfindung betrifft ein Operationswerkzeug, insbesondere zum Einlegen einer Bauchhöhlendrainage, bestehend aus einer Greifzange mit zwei Schenkeln, die einerseits Klemmbacken und andererseits Griffe bilden, und mit einem in dieser längsverschieblich geführten, als Stichinstrument dienenden Dorn.

In der heutigen operativen Medizin werden Drainagen an offenen Bauchhöhlen mittels einer Operationsschere und einer Greifzange eingelegt. Das Einlegen gliedert sich dabei grob in zwei getrennte Vorgänge auf. Zunächst arbeitet sich der Operateur mit der Operationsschere mühsam durch die Bauchdecke bis in die offene Bauchhöhle vor. Dabei führt er mit der Operationsschere schneidende Bewegungen aus, wobei er die Schere bei jedem Öffnen wieder bereichsweise aus dem Stichkanal herausziehen muß. Danach fixiert der Operateur mit einem Finger die innere Öffnung des Stichkanals in der Bauchhöhle, indem er den Finger an die Spitze der Operationsschere führt, und zieht dann die Operationsschere heraus. In dem zweiten Arbeitsschritt wird die Greifzange, z. B. eine Kornzange, von außen durch den Stichkanal durch alle Schichten auf den Finger des Operateurs zu in die offene Bauchhöhle geführt. Sobald die Greifzange durch den Stichkanal geführt ist, muß diese gegen den Widerstand des Gewebes kraftaufwendig geöffnet werden, damit der Drainageschlauch gegriffen und durch die Schichten der Bauchdecke hindurchgezogen werden kann.

Diese Technik des Einlegens einer Bauchhöhlendrainage bringt durch den in zwei Verfahrensschritte unterteilten Vorgang eine Vielzahl von Problemen mit sich. Aufgrund der als Stichwerkzeug ungeeigneten Form der Operationsschere ist das Durchstechen der Bauchdecke mühsam. Durch das ständige Öffnen und Zurückziehen der Operationsschere kann es dabei sowohl zu einer Traumatisierung der verschiedenen Gewebeschichten der Bauchdecke als auch zu Blutungen kommen. Da nach dem Durchstechen der Operationsschere der Stichkanal mit dem Finger fixiert wird, besteht zum einen die Gefahr einer Verletzung der Fingerspitze des Operateurs durch die Spitze der Operationsschere und zudem die Gefahr einer unzulässigen Aufweitung der Punktionsöffnung durch den Finger des Operateurs. Ferner ist das Einführen der Greifzange ein problematischer Arbeitsschritt, da diese durch den bereits vorliegenden Stichkanal geführt werden muß. Hierdurch kann es in der Praxis zu einer zusätzlichen Gewebetraumatisierung kommen, wenn aufgrund des weichen Gewebes der Stichkanal mit der Greifzange nicht sofort gefunden wird. Auch das spätere Greifen des Drainageschlauches mit der Greifzange in der offenen Bauchhöhle ist mühsam, da das Öffnen der Greifzange durch die ihre Greifschenkel umgebende Bauchdecke erschwert wird.

Aus der WO 94/05224 ist ein Operationswerkzeug gemäß dem Oberbegriff des Anspruchs 1 zur Entnahme von Gewebe bekannt, das zwei längs zueinander verschiebliche Schenkel aufweist. An dem patientennahen Ende sind die Schenkel gegenüber der Verschieberichtung abgewinkelt und als Backen ausgebildet. Im Bereich der Backen ist ein längsverschieblicher Dorn geführt, der im Bereich der Abwinklung durch eine Bohrung aus einem der Schenkel austritt. Bei der Benutzung des Operationswerkzeuges muß der Operateur dieses mit der einen Hand an dem patientennahen Ende, damit die Dornspitze beim Einstechen endseitig aus den Backen hervorragt und ein Verdrehen des Dornes beim Einstechen verhindert wird, und mit der anderen Hand an dem anderen Ende festhalten, um das Operationswerkzeug auszurichten. Nach Durchstoßen muß der Dorn aus dem Operationswerkzeug vollständig gezogen werden, damit die Backen betätigbar sind.

Aufgabe der Erfindung ist es, ein Operationswerkzeug anzugeben, das die verschiedenen Arbeitsschritte bei lediglich einmaligem Durchstoßen der Bauchdecke ermöglicht. Zudem soll das Ergreifen des Drainageschlauches vereinfacht werden.

Diese Aufgabe wird dadurch gelöst, daß die Schenkel durch einen Gelenkbolzen, wobei der Gelenkbolzen der Greifzange eine Bohrung als Lager für den Dorn aufweist, verbunden sind. Aufgrund dieser Anordnung ist das Stich- als auch das Greifinstrument als stabile Einheit in einem Operationswerkzeug zusammengefaßt, das die Funktionen Stechen, Greifen und das anschließende Ziehen des Drainageschlauches ermöglicht. Zunächst wird das Operationswerkzeug mit der scharfen Spitze des Dornes unter Mitnahme der Greifzange leicht und gewebeschonend nach einer Inzision durch die Bauchdecke gestochen. Bei Durchtritt der Spitze des Operationswerkzeuges in die offene Bauchhöhle wird der Dorn in dem Operationswerkzeug zurückgezogen, so daß der Drainageschlauch nach dem Öffnen der Greifzange von dieser ergriffen und durch die Bauchdecke gezogen werden kann.

Zur Führung des Dorns in der Greifzange weist der Gelenkbolzen der Greifzange eine Bohrung als Lager für den Dorn auf. Hierdurch wird der Dorn von den beiden Schenkeln der Greifzange umfaßt, so daß beim Einführen des Operationswerkzeuges in die Bauchdecke der Dorn von beiden Schenkeln gestützt wird.

Zur verbesserten Fixierung und Führung des Dornes weisen die Klemmbacken der Greifzange Führungsnuten für den Dorn auf, so daß der Dorn im Bereich der Klemmbacken auf ganzer Länge geführt wird.

Zum verbesserten Durchstechen der Bauchdecke mit dem Operationswerkzeug ist die Spitze des Dornes kegelförmig mit nach außen gekrümmter Oberfläche ausgebildet und geht in die ebenfalls gekrümmte Außenkontur der Klemmbacken der Greifzange über. Dadurch erhält die Spitze des Operationswerkzeuges eine glatte Oberfläche, so daß das Durchstechen der Bauchdecke leicht und gewebeschonend durchführbar ist. Gewebeverletzungen werden aufgrund der Keilform nahezu ausgeschlossen.

Zweckmäßigerweise kann sich die Bohrung beidseitig zur Mantelfläche des Gelenkbolzens hin erweitern. Hierdurch wird zum einen der Dorn in der Bohrung des Gelenkbolzens geführt, aber zum anderen dem Dorn ein gewisser Spielraum gelassen, so daß ein exaktes Ausrichten des Dornes bei geöffneter Greifzange möglich ist.

Bei einem erfindungsgemäßen Operationswerkzeug können die Führungsnuten eine Verengung aufweisen, in der der Dorn mit einer Verjüngung verschieblich geführt ist und die bei zurückgezogenem Dorn als Auflager für die gegenüber der Verjüngung dickere Spitze des Dorns zum Offenhalten der Greifzange dient. Durch diese Konstruktion sowohl der Führungsnuten als auch des Dornes wird das Öffnen der Greifzange erleichtert, wenn diese bereits durch die Bauchdecke gestoßen ist und der in der offenen Bauchhöhle befindliche Drainageschlauch gegriffen werden soll. Während beim Durchstoßen der Greifzange mit innenliegendem Dorn die Verjüngung des Dornes im Bereich der Verengung der Führungsnuten liegt, kommt es beim Herausziehen des innenliegenden Dornes zu einem Spreizen der Schenkel der Greifzange, da der sich zur Spitze hin verdickende Dorn mit seinem verdickten Bereich auf die Erhebung der Führungsnuten gleitet.

Um das Herausziehen des Dornes und somit das Spreizen der Greifzange bei in die Bauchdecke eingeführtem Operationswerkzeug zu erleichtern, kann der Dorn im Bereich der Griffe der Greifzange eine Halteöse aufweisen. Hieran kann der Dorn sicher gegriffen und herausgezogen werden. Ferner bietet sich die Möglichkeit, die Halteöse, sofern eine Arretierung an den Griffen der Greifzange vorhanden ist, an dieser zu befestigen, so daß der Dorn beim Durchstechen der Bauchdecke gesichert ist.

Um auch das Durchstechen an schwer zugänglichen Stellen oder aber auch eine nachträgliche Korrektur des Stichkanals zu ermöglichen, können die Greifzange und der innenliegende Dorn über die Länge des Operationswerkzeuges gekrümmt ausgebildet sein.

Im folgenden werden ein in der Zeichnung dargestelltes Ausführungsbeispiel der Erfindung erläutert; es zeigt:

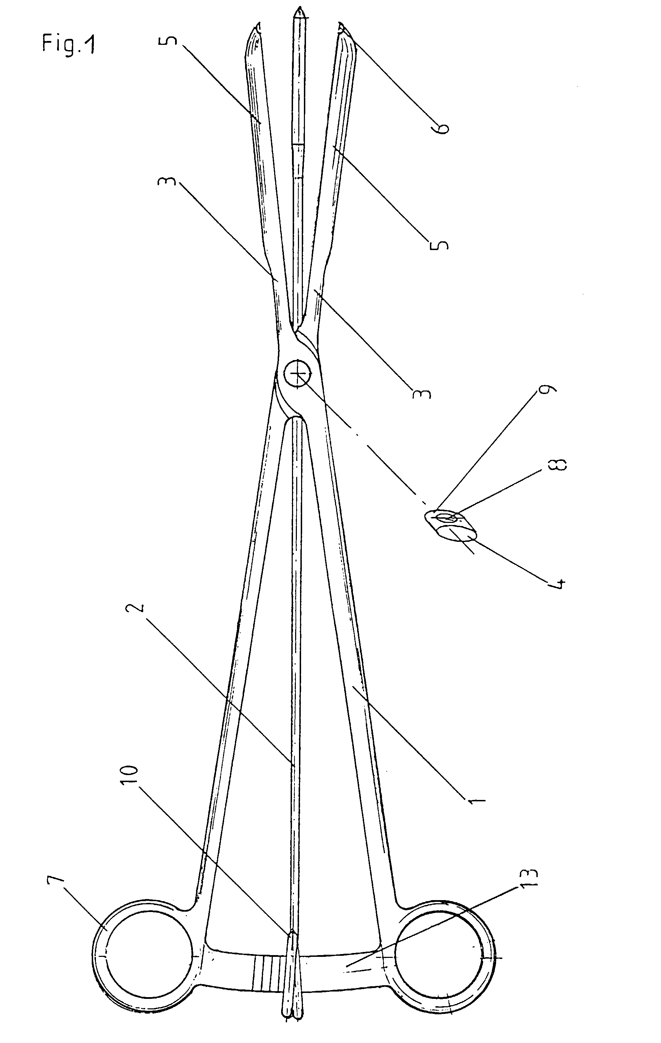

- Fig. 1: eine Draufsicht auf eine geöffnete Greifzange mit innenliegendem Dorn.

Fig. 1 zeigt ein Operationswerkzeug bestehend aus einer Greifzange 1 und einem innenliegenden Dorn 2. Die Greifzange 1 besteht aus Schenkeln 3, die über einen Gelenkbolzen 4 , der aus Vereinfachungsgründen neben dem Werkzeug dargestellt ist, beweglich miteinander verbunden sind. Auf der einen Seite der Greifzange 1 sind an den Schenkeln 3 Klemmbacken 5 mit innenliegenden Führungsnuten 6 angeformt. An den den Klemmbacken 5 gegenüberliegenden Enden der Greifzange 1 sind Griffe 7 zum Halten der Greifzange 1 vorgesehen.

Zwischen den beiden Schenkeln 3 der Greifzange 1 ist der Dorn 2 angeordnet, der in einer Bohrung 8 in dem Gelenkbolzen 4 verschieblich gelagert ist. Die Bohrung 8 erweitert sich dabei beidseitig zur Mantelfläche 9 des Gelenkbolzens 4. Im eingeklemmten Zustand des Dornes 2 zwischen den Klemmbacken 5 der Greifzange 1 geht die kegelförmig mit nach außen gekrümmter Oberfläche ausgebildete Spitze des Dornes 2 in die ebenfalls gekrümmte Außenkontur der Klemmbacken 5 über. An den der Spitze des Dornes 2 gegenüberliegenden Enden ist eine Halteöse 10 angeformt.

Die Führungsnuten 6 der Klemmbacken 5 bilden eine Verengung. Im Bereich der Verengung weist der Dorn 2 eine Verjüngung auf. Gegenüber dieser Verjüngung verdickt sich der Dorn 2 wieder in Richtung auf seine Spitze hin. Beim Zurückziehen des Dornes 2 gleitet dieser mit seinem verdickten vorderen Bereich auf die Verengung der Klemmbacken 5, wodurch die Schenkel 3 der Greifzange 1 gespreizt werden.

Zur Fixierung des Dornes 2 ist an den Griffen 7 der Greifzange 1 eine Arretierung 13 vorgesehen, die bei geschlossenen Klemmbacken 5 die Halteöse 10 des Dornes 2 durchgreift.

Bei einer anderen Ausführungsform ist die Greifzange 1 mit angeformten Griffen 7 und einem innenliegenden Dorn 2 gekrümmt. An dem der Spitze des Dornes 2 gegenüberliegenden Ende ist die Halteöse 10.

## Patentansprüche

1. Operationswerkzeug, insbesondere zum Einlegen einer Bauchhöhlendrainage, bestehend aus einer Greifzange (1) mit zwei Schenkeln (3), die einerseits Klemmbacken (5) und andererseits Griffe (7) bilden, und mit einem in dieser längsverschieblich geführten, als Stichinstrument dienenden Dorn (2), wobei die Klemmbacken (5) der Greifzange (1) Führungsnuten (6) für den Dorn (2) aufweisen, und wobei die Spitze des Dornes (2) kegelförmig mit nach außen gekrümmter Oberfläche ausgebildet ist und in die ebenfalls gekrümmte Außenkontur der Klemmbacken (5) der Greifzange (1) übergeht dadurch gekennzeichnet, daß die Schenkel (3) durch einen Gelenkbolzen (4), wobei der Gelenkbolzen (4) der Greifzange (1) eine Bohrung (8) als Lager für den Dorn (2) aufweist, verbunden sind.

2. Operationswerkzeug nach Anspruch 1, dadurch gekennzeichnet, daß sich die Bohrung (8) beidseitig zur Mantelfläche (9) des Gelenkbolzens (4) hin erweitert.

3. Operationswerkzeug nach Anspruch 1 oder 2, dadurch gekennzeichnet, daß die Führungsnuten (6) eine Verengung aufweisen, in der der Dorn (2) mit einer Verjüngung verschieblich geführt ist und die bei zurückgezogenem Dorn (2) als Auflager für die gegenüber der Verjüngung dickeren Spitze des Dornes (2) zum Offenhalten der Greifzange (1) dient.

4. Operationswerkzeug nach einem der Ansprüche 1 bis 3, dadurch gekennzeichnet, daß der Dorn (2) im Bereich der Griffe (7) der Greifzange (1) eine Halteöse (10) aufweist.

5. Operationswerkzeug nach einem der Ansprüche 1 bis 4, dadurch gekennzeichnet, daß die Greifzange (1) und der innenliegende Dorn (2) über die Länge des Operationswerkzeuges gekrümmt ausgebildet sind.

## Claims

1. Operating instrument, more especially for introducing an abdominal cavity drainage, comprising gripping tongs (1) provided with two portions (3), which form clamping jaws (5) at one end and handles (7) at the other end, and provided with a spindle (2), which is longitudinally displaceably guided in said tongs and serves as a piercing instrument, the clamping jaws (5) of the gripping tongs (1) having guide grooves (6) for the spindle (2), and the tip of the spindle (2) having a conical configuration with an outwardly curved surface and extending into the equally curved external configuration of the clamping jaws (5) of the gripping tongs (1), characterised in that the portions (3) are connected by a hinge pin (4), the hinge pin (4) of the gripping tongs (1) having a bore (8) as the bearing for the spindle (2).

2. Operating instrument according to claim 1, characterised in that the bore (8) extends on both sides towards the surface (9) of the hinge pin (4).

3. Operating instrument according to claim 1 or 2, characterised in that the guide grooves (6) have a constriction, in which the spindle (2) is displaceably guided with a tapering, and, when the spindle (2) has been withdrawn, said constriction serves as a support for the tip of the spindle (2), which is thicker than the tapering, to keep the gripping tongs (1) open.

4. Operating instrument according to one of claims 1 to 3, characterised in that the spindle (2) has a retaining lug (10) in the region of the handles (7) of the gripping tongs (1).

5. Operating instrument according to one of claims 1 to 4, characterised in that the gripping tongs (1) and the internally situated spindle (2) have a curved configuration over the length of the operating instrument.

## Revendications

1. Outil médical d'opération, notamment pour mettre en place un drain de cavité abdominale, constitué d'un davier (1) à deux branches (3) qui forment à une extrémité des mors (5) et à l'autre extrémité des poignées (7), et avec un poinçon (2), servant d'instrument perceur, guidé en coulissement longitudinal dans ce davier, les mors (5) du davier (1) présentant des rainures de guidage (6) pour le poinçon (2) et la pointe du poinçon (2) étant réalisée conique avec une surface courbée vers l'extérieur, qui se raccorde au contour extérieur également courbe des mors (5) du davier (1),
**caractérisé** en ce que les branches (3) sont assemblées par un boulon d'articulation (4), le boulon d'articulation (4) du davier (1) présentant un perçage (8) comme palier pour le poinçon (2).

2. Outil médical d'opération selon la revendication 1, **caractérisé** en ce que le perçage (8) s'élargit de part et d'autre en direction de la face d'enveloppe (9) du boulon d'articulation (4).

3. Outil médical d'opération selon la revendication 1 ou 2, **caractérisé** en ce que les rainures de guidage (6) présentent un rétrécissement, dans lequel le poinçon (2) est guidé en coulissement par une partie rétrécie et qui sert, lorsque le poinçon (2) est retiré, d'appui pour la pointe du poinçon (2), plus épaisse que la partie rétrécie, afin de maintenir le davier (1) ouvert.

4. Outil médical d'opération selon une des revendications 1 à 3, **caractérisé** en ce que le poinçon (2) présente un oeillet de retenue (10) dans la région des poignées (7) du davier (1).

5. Outil médical d'opération selon une des revendications 1 à 4, **caractérisé** en ce que le davier (1) et le poinçon (2) qui se trouve à l'intérieur sont réalisés courbes sur la longueur de l'outil médical d'opération.
